# EUROPÄISCHE PATENTANMELDUNG

(11) **EP 1 197 206 A1**
(43) Veröffentlichungstag der Anmeldung: **17.04.2002**
(21) Anmeldenummer: 01119973.4
(22) Anmeldetag: 18.08.2001
(51) Int. Cl.: A61K 7/50, C11D 1/62, C11D 3/30

(54) **Verwendung von Betainestern als Verdickungsmittel**

(30) Priorität: 29.08.2000 DE 10042374
(71) Anmelder: Cognis Deutschland GmbH, 40589 Düsseldorf (DE)
(72) Erfinder: Rathjens, Andreas, 40589 Düsseldorf (DE); Uphues, Günter, 40789 Monheim (DE); Nieendick, Claus, 47807 Krefeld (DE)

(57) **Zusammenfassung**

Vorgeschlagen wird die Verwendung von Betainestern, die durch Veresterung von Fettalkoholen und/oder Polyolen mit Halogencarbonsäuren oder deren Salzen und anschliessender Quaternisierung der resultierenden Mischung mit tertiären Aminen erhältlich sind, als Verdickungsmittel in oberflächenaktiven Zubereitungen.

## Beschreibung

### Gebiet der Erfindung

Die Erfindung betrifft die Verwendung von Betainestern als Verdickungsmittel für oberflächenaktive Zubereitungen.

### Stand der Technik

Oberflächenaktive Zubereitungen, z.B. wäßrige tensidhaltige Zubereitungen, wie beispielsweise Haarshampoos oder Reinigungsmittel, weisen häufig eine unzureichend niedrige Viskosität auf. Es ist sofort klar, dass sich ein Mittel, welches ein Fließverhalten wie Wasser aufweist, nur schwer dosieren lässt und damit für den Verbraucher die Gefahr besteht, dass er stets mehr einsetzt als gewünscht. Um dieses Problem zu umgehen, werden solchen Zubereitungen Verdickungsmittel zugesetzt, bei denen es sich häufig um synthetische polymere Verbindungen, wie beispielsweise Poly(meth)acrylate, Polyvinylverbindungen, Polycarboxylate, abgewandelte Naturstoffe, wie z.B. Carboxymethylcellulosen und Celluloseether oder auch natürliche Produkte, z.B. Agar-Agar, Carrageen, Alginate, Pektine, aber auch Polykieselsäuren oder Tonmineralien handeln kann. Allen diesen ist gemein, dass sie außer der verdickenden Wirkung keinen Beitrag zum Leistungsvermögen der Formulierungen liefern. Dementsprechend sollten Mittel gefunden werden, die nicht nur verdickende Eigenschaften aufweisen, sondern darüber hinaus weitere Eigenschaften aufweisen, beispielsweise tensidische Eigenschaften sowie gute biologische Abbaubarkeit.

Die Aufgabe der Erfindung hat folglich darin bestanden, Stoffe zur Verfügung zu stellen, die ein möglichst breites Spektrum von oberflächenaktiven Zubereitungen zuverlässig verdicken, gleichzeitig selbst über tensidische Eigenschaften verfügen und gut biologisch abbaubar sind.

### Beschreibung der Erfindung

Gegenstand der Erfindung ist die Verwendung von Betainestern, dadurch erhältlich, dass man Fettalkohole und/oder Polyole mit Halogencarbonsäuren oder deren Salzen verestert und die resultierende Mischung mit tertiären Aminen quaternisiert, als Verdickungsmittel in oberflächenaktiven Zubereitungen.

Überraschenderweise wurde gefunden, dass sich Betaintenside, die durch Umsetzung von Polyolen und/oder Fettalkoholen mit Halogencarbonsäuren und anschliessender Quaternisierung der resultierenden Mischung erhalten werden, als Verdickungsmittel für oberflächenaktive Zubereitungen, vorzugsweise solche auf Tensidbasis, eignen. Darüber hinaus zeigen diese Mittel tensidische Eigenschaften und sind aufgrund ihrer Struktur gut biologisch abbaubar. Ebenso wird die Solubilisierbarkeit hydrophober Formulierungsbestandteile begünstigt.

### Polyole

Polyole, die im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Vorzugsweise werden als Polyole Glycerin, Diglycerin, Trimethylolpropan, Pentaerythrit, Sorbit, Propylenglycol, Butylenglycol, Hexylenglycol sowie Polyethylenglycole mit einem durchschnittlichen Molekulargewicht im Bereich von 100 bis 1.000 Dalton eingesetzt. In einer besonderen Ausführungsform der Erfindung werden als Polyole Niedrigalkylglucoside, Zuckeralkohole und Zucker, beispielsweise Sorbit, eingesetzt.

### Fettalkohole

Unter Fettalkoholen sind primäre aliphatische Alkohole der Formel **(I)** zu verstehen,

R¹O(CH₂CH₂O)ₘH (I)

in der R¹ für einen linearen oder verzweigten, gesättigten oder ungesättigten, vorzugsweise verzweigten gesättigten oder ungesättigten und insbesondere verzweigten, gestättigten Kohlenwasserstoffrest mit 6 bis 22, vorzugsweise 8 bis 18 und insbesondere 10 bis 16 Kohlenstoffatomen und m für Zahlen von durchschnittlich 0 bis 10, vorzugsweise 2 bis 6 und insbesondere 4 steht.

Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

### Betainester

Die erfindungsgemäßen Betainester werden durch Veresterung von Fettalkoholen und/oder Polyolen mit Halogencarbonsäuren, vorzugsweise Halogenessigsäuren und insbesondere Chloressigsäure, unter Entzug des entstehenden Reaktionswassers und nachfolgender Quaternisierung des Reaktionsgemisches mit tertiären Aminen, vorzugsweise N,N'-Dimethyl-N-Alkylaminen, erhalten. Die Entfernung des Reaktionswassers erfolgt vorteilhafterweise mittels Wasserschleppmitteln wie Toluol, Xylol oder Anlegen eines Unterdrucks von vorzugsweise 100 mbar. Die Quaternisierung wird bei 80 bis 85 °C durchgeführt, bis der Gehalt an restlichem Aminstickstoff < 0,2, vorzugsweise < 0,1 % beträgt. Eventuell kann das Wasserschleppmittel schon vorher mittels Rotationsverdampfer entfernt werden.

In einer bevorzugten Ausführungsform der Erfindung werden der Fettalkohol und/oder das Polyol mit der Halogenessigsäure in einer Rührapparatur mit Rückflusskühler und einem Wasserabscheider nach Dean-Stark bei Anwesenheit eines Wasserschleppmitteln, vorzugsweise Toluol und Xylol, unter Rückfluss erhitzt bis die berechnete Menge Wasser abgeschieden wurde, vorzugsweise bis die Säurezahl kleiner 1 mg/g KOH ist. Die wasserschleppmittelenthaltende Lösung wird anschließend in einer zweiten Rührapparatur vorgelegt und bei 80 bis 85 °C das tertiäre Amin zugetropft. Zur Vervollständigung der Reaktion wird bei gleicher Temperatur noch 4 bis 8, vorzugsweise 5 bis 6,5 Stunden nachgerührt, bis der Gehalt an titrierbarem Stickstoff auf unter 0,2, vorzugsweise 0,1 % abgesunken ist. Unter Verwendung eines Rotationsverdampfers wird das enthaltene Wasserschleppmittel destillativ entfernt und der Rückstand mit Wasser zu einer klaren, vorzugsweise 30 bis 60 und insbesondere 40 bis 50 Gew.-%igen Lösung verdünnt.

Exemplarisch werden im folgenden einige Beispiele für Betainester angegeben:

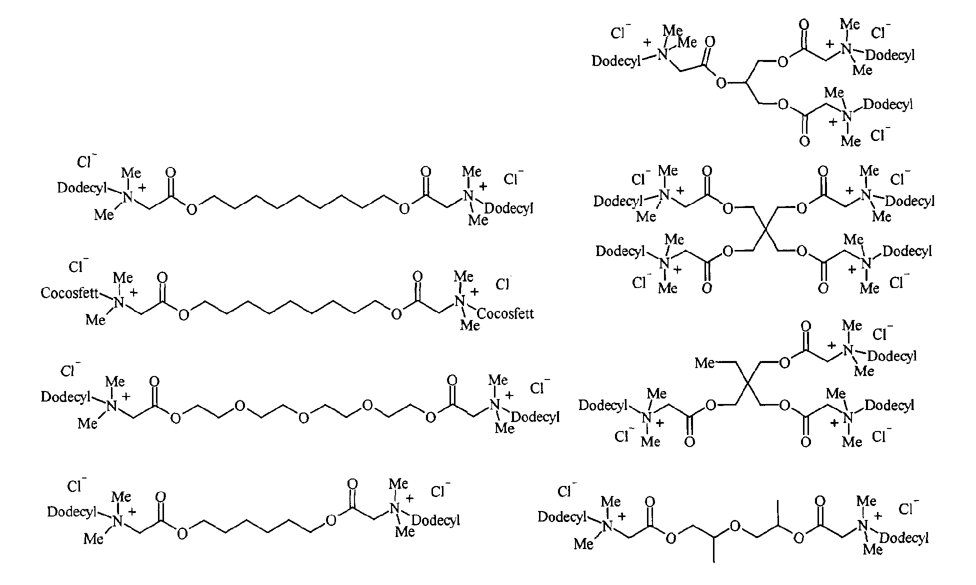

Üblicherweise werden die Betainester als Verdickungsmittel in Mengen von 0,1 bis 15, vorzugsweise 0,5 bis 7 und insbesondere 2 bis 5 Gew.-% - bezogen auf den Feststoffgehalt der oberflächenaktiven Zubereitungen, eingesetzt.

Unter Verwendung der erfindungsgemässen Betainester können oberflächenaktive Zubereitungen, vorzugsweise wässrige oberflächenaktive Zubereitungen auf Basis von Tensiden, erhalten werden, die Viskositäten gemessen nach der Brookfield-Methode in einem DV II-Viskosimeter (23 °C, Spindel 4 oder 5, 10 rpm) von 2000 bis 40000, vorzugsweise 10000 bis 24000 und insbesondere 15000 bis 20000 CPS aufweisen.

Besonders verdickende Wirkung von oberflächenaktiven Zubereitungen zeigen Betainester, die durch Umsetzung von verzweigen Polyolen, wie beispielsweise Pentaerythrit, Diglycerin und Trimethylopropan sowie komplexeren Polymeren auf Basis von Zuckern, wie beispielsweise Sorbit, mit Chloressigsäure und tert-Aminen, vorzugsweise N,N-Dimethyl-N-Alkylaminen, gebildet werden.

### Tensidlösungen

Ein besonderer Vorteil der Erfindung besteht darin, dass unter Zusatz der erfindungsgemässen Betainester eine Vielzahl oberflächenaktiver Zubereitungen (vgl. Kapitel Hilfs- und Zusatzstoffe), vorzugsweise wässrige oberflächenaktive Zubereitungen auf Basis von Tensiden, vorzugsweise anionischen und/oder amphoteren bzw. zwitterionischen Tensiden, zuverlässig verdickt werden können. Durch die Verwendung der Betainester wird der Tensidanteil in der Formulierung erhöht, so dass die eingesetzte Tensidmenge um den Anteil des Betainesters vermindert werden kann, ohne dass sich die Eigenschaften der Zubereitungen ändern.

Typische Beispiele für Tenside, deren wäßrige Lösungen durch den Zusatz der Betainester verdickt werden können, sind **anionische Tenside,** wie z.B. Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind gesättigte Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysato** **ren, Tenside und Mineralöladditive", Thieme Verlag, Stuttgart, 1978, S. 123-217** verwiesen. Vorzugsweise werden wäßrige Lösungen von Alkylethersulfaten, Monoglycerid(ether)sulfaten, Alk(en)yloligoglykosiden sowie gesättigten Betainen verdickt. Die Feststoffkonzentration in den Lösungen kann dabei 5 bis 60 und vorzugsweise 10 bis 50 und insbesondere 20 bis 40 Gew.-% betragen.

### Hilfs- und Zusatzstoffe

Unter oberflächenaktiven Zubereitungen sind im Sinne der Erfindung Wasch-, Spül-, Reinigungs- und Avivagemittel sowie kosmetische und/oder pharmazeutische Zubereitungen, wie beispielsweise Haarshampoos, Haarlotionen, Schaumbäder, Duschbäder, Mund- und Zahnpflegemittel, Cremes, Gele, Lotionen, alkoholische und wäßrig/alkoholische Lösungen, Emulsionen, Wachs/ Fett-Massen, Stiftpräparate, zu verstehen. Die zu verdickenden oberflächenaktiven Zubereitungen können ferner als weitere Hilfs- und Zusatzstoffe Ölkörper, Emulgatoren, Perlglanzwachse, Konsistenzgeber, zusätzliche Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

Sofern die erfindungsgemäßen Stoffe zur Verdickung von Wasch-, Spül-, Reinigungs- oder Avivagemitteln ("Softener") eingesetzt werden, können weitere typische Inhaltsstoffe, wie beispielsweise Builder, Bleichmittel, Bleichaktivatoren, Lösungsmittel, Waschkraftverstärker, Enzyme, Enzymstabilisatoren, Viskositätsregulatoren, Vergrauungsinhibitoren, optische Aufheller, Soil repellants, Schauminhibitoren, anorganische Salze sowie Duft- und Farbstoffe enthalten sein.

Der Gesamtanteil der Hilfs- und Zusatzstoffe an den oberflächenaktiven Zubereitungen kann 5 bis 99 und vorzugsweise 10 bis 80 und insbesondere 20 bis 65 Gew.-% - bezogen auf den Feststoffgehalt der oberflächenaktiven Zubereitungen - betragen. Die Herstellung der oberflächenaktiven Zubereitungen kann durch übliche Kalt - oder Heißprozesse erfolgen; vorzugsweise arbeitet man nach der Phaseninversionstemperatur-Methode.

### Beispiele

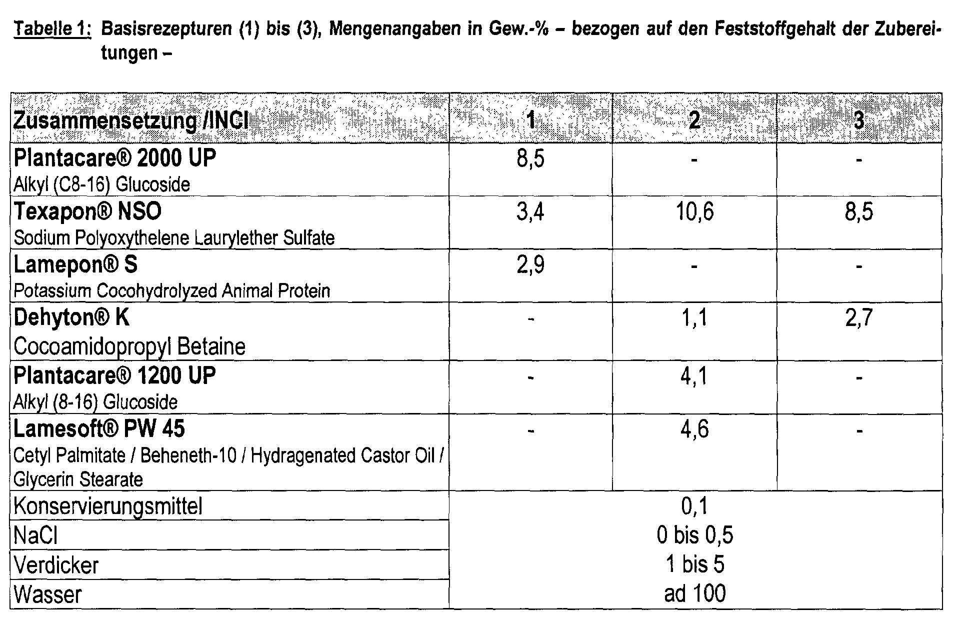

Die in Tabelle 1 aufgeführten Tensidlösungen 1, 2 und 3 können 1 bis 5 Gew.-% Verdicker enthalten. In Tabelle 2 werden einige Beispiele aufgeführt, die 2 bis 5 Gew.-% unterschiedlicher Betainester als Verdicker enthalten. Zur Bezeichnung der eingesetzten Betainester wurde jeweils angegeben, über welche Ausgangsstoffe diese erhalten wurden. Die Viskositäten der Tensidlösungen 1 bis 3 wurden nach der Brookfield-Methode in einem DV II-Viskosimeter (24°C, Spindel 4, 10 rpm, CPS) untersucht. Die Ergebnisse sind in Tabelle 2 zusammengefasst.

### Herstellungsbeispiel 1 eines Betainesters

183,3 g Decandiol-1,10, 95 % (1 Mol), 190,9 g Chloressigsäure (2 Mol) und 300 ml Toluol werden in einer Rührapparatur mit Rückflusskühler und einem Wasserabscheider nach Dean-Stark solange zum Rückfluss erhitzt, bis 36 g Wasser abgeschieden sind. Die Säurezahl war kleiner 1 mg/g KOH.
281,4 g der toluolischen Lösung (0,45 Mol) werden anschließend in einer zweiten Rührapparatur vorgelegt. Bei 80 bis 85 °C werden danach 200,1 g Dimethyldodecylamin (6,3 % N-titrierbar) zugetropft. Zur Vervollständigung der Reaktion wird bei gleicher Temperatur noch 6,5 Stunden nachgerührt, bis der Gehalt an titrierbarem Stickstoff auf unter 0,2 % abgesunken ist. Unter Verwendung eines Rotationsverdampfers wird das Toluol destillativ entfernt und der Rückstand (374,5 g) mit der gleichen Menge Wasser zu einer klaren, 50 %igen Lösung verdünnt.
Die Herstellung der weiteren in Tabelle 2 aufgeführten Betainester erfolgt analog.

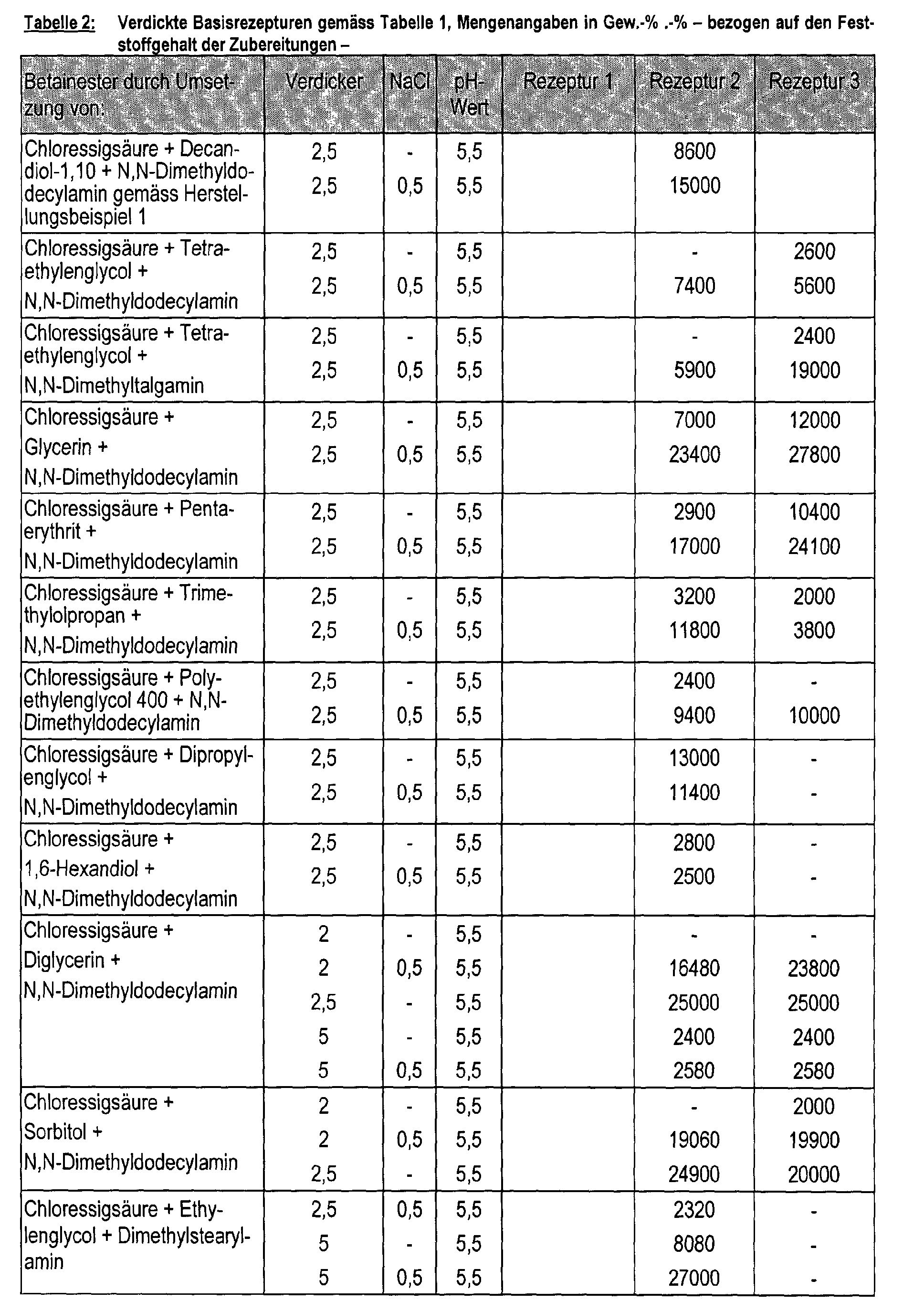

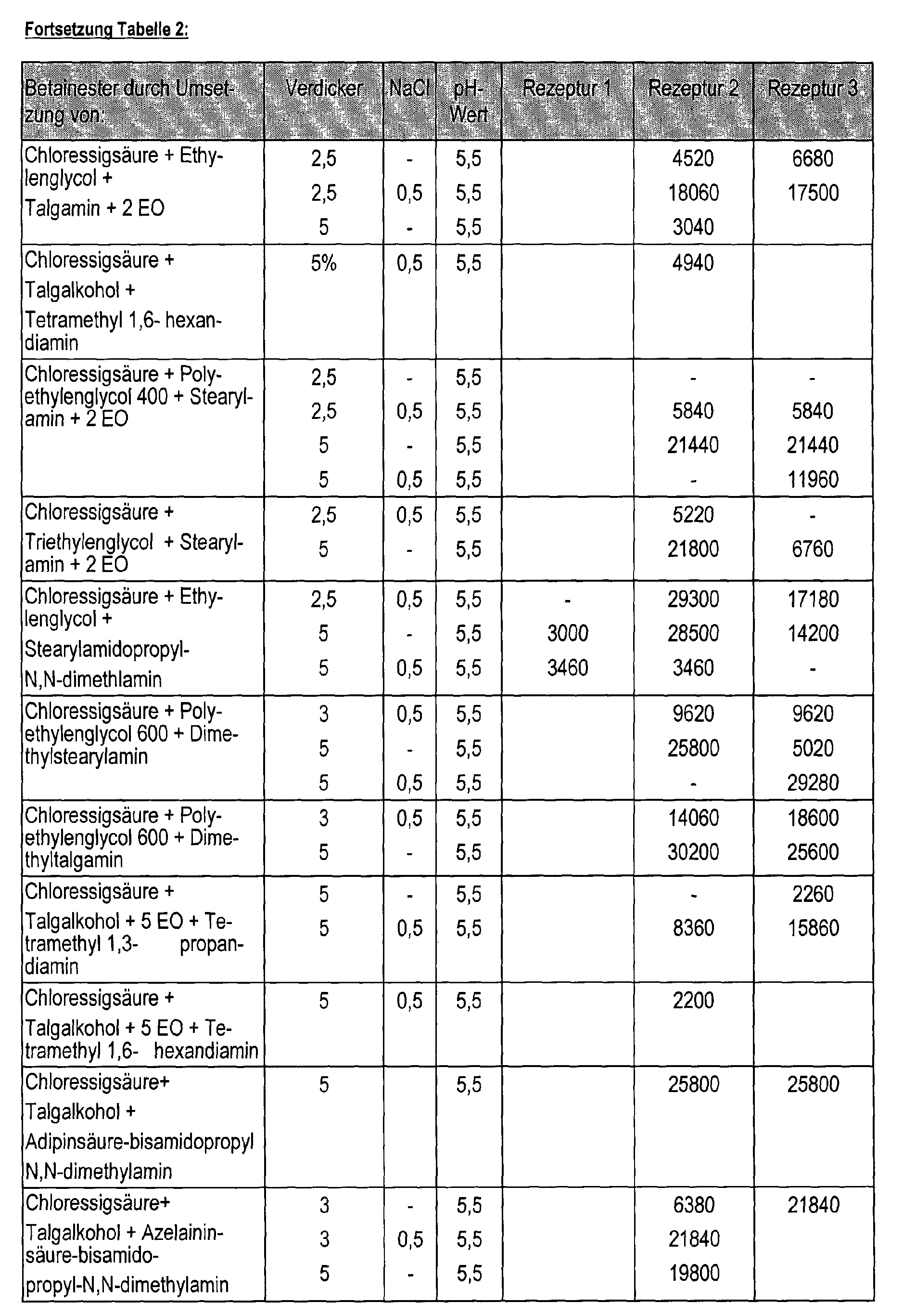

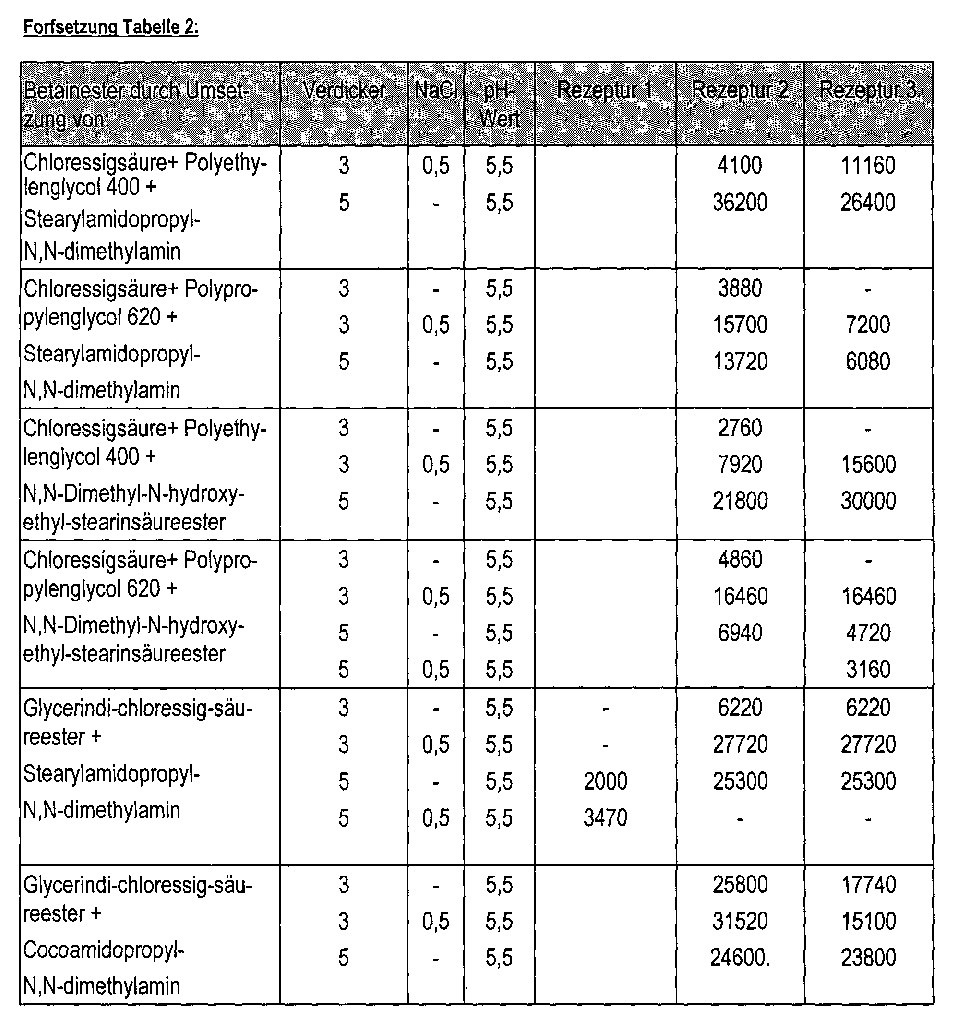

## Patentansprüche

1. Verwendung von Betainestern, **dadurch erhältlich,** dass man Fettalkohole und/oder Polyole mit Halogencarbonsäuren oder deren Salzen verestert und die resultierende Mischung mit tertiären Aminen quaternisiert, als Verdickungsmittel in oberflächenaktiven Zubereitungen.

2. Verwendung nach Anspruch 1, **dadurch gekennzeichnet, dass** man Polyole einsetzt, die ausgewählt sind aus der Gruppe, die gebildet wird von Glycerin, Alkylenglycolen, technischen Oligoglyceringemischen, Methylolverbindungen, Niedrigalkylglucosiden, Zuckeralkoholen, Zuckern, Aminozuckern und Dialkoholaminen.

3. Verwendung nach den Ansprüchen 1 und/oder 2, **dadurch gekennzeichnet, dass** man Polyole einsetzt, die ausgewählt sind aus der Gruppe, die gebildet wird von Diglycerin, Trimethylolpropan, Pentaerythrit, Sorbit, Propylenglycol, Butylenglycol, Hexylenglycol sowie Polyethylenglycole mit einem durchschnittlichen Molekulargewicht im Bereich von 100 bis 1.000 Dalton.

4. Verwendung nach Anspruch 1, **dadurch gekennzeichnet, dass** man Fettalkohole der Formel **(I)** einsetzt,
R¹O(CH₂CH₂O)ₘH (I)
in der R¹ für einen linearen oder verzweigten, gesättigten oder ungesättigten Kohlenwasserstoffrest mit 6 bis 22 Kohlenstoffatomen und m für Zahlen von durchschnittlich 0 bis 10 steht.

5. Verwendung nach mindestens einem der Ansprüche 1 bis 4, **dadurch gekennzeichnet, dass** man Halogenessigsäuren einsetzt.

6. Verwendung nach mindestens einem der Ansprüche 1 bis 5, **dadurch gekennzeichnet, dass** man die Betainester in Mengen von 0,1 bis 15 Gew.-% - bezogen auf den Feststoffgehalt der oberflächenaktiven Zubereitungen - einsetzt.

7. Verwendung nach mindestens einem der Ansprüche 1 bis 6, **dadurch gekennzeichnet, dass** man wässrige oberflächenaktive Zubereitungen von anionischen, nichtionischen, kationischen und/oder amphoteren bzw. zwitterionischen Tensiden verdickt.

8. Verwendung nach mindestens einem der Ansprüche 1 bis 7, **dadurch gekennzeichnet, dass** man oberflächenaktive Zubereitungen verdickt, die einen Feststoffgehalt im Bereich von 5 bis 99 Gew.-% aufweisen.
